(19) Europäisches Patentamt
European Patent Office
Office européen des brevets

(11) **EP 3 964 194 A1**

(12) **EUROPEAN PATENT APPLICATION**

(43) Date of publication:
**09.03.2022 Bulletin 2022/10**

(21) Application number: **21397509.7**

(22) Date of filing: **07.09.2021**

(51) International Patent Classification (IPC):
**A61K 8/9789** (2017.01)    **A61Q 17/04** (2006.01)
**A61Q 19/08** (2006.01)

(52) Cooperative Patent Classification (CPC):
**A61Q 17/04; A61K 8/9789; A61Q 19/08;**
A61Q 1/02; A61Q 1/06; A61Q 1/10; A61Q 19/10

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.09.2020   FI 20205864**

(71) Applicant: **Lumene Oy**
**02780 Espoo (FI)**

(72) Inventors:
• **ISOHANNI, Tiina**
**02780 Espoo (FI)**
• **MÄKINEN, Päivi**
**02780 Espoo (FI)**
• **PESONEN, Terhi**
**02780 Espoo (FI)**
• **PALMUJOKI, Ingela**
**02780 Espoo (FI)**
• **VÄNTTINEN, Kristofer**
**02780 Espoo (FI)**
• **SAHRAMO, Elina**
**02780 Espoo (FI)**

(74) Representative: **Laine IP Oy**
**Porkkalankatu 24**
**00180 Helsinki (FI)**

(54) **COSMETIC COMPOSITION AND METHOD FOR PROTECTING SKIN AND KERATIN FIBRES FROM HIGH ENERGY VISIBLE LIGHT**

(57)    According to an example aspect of the present invention, there is provided a cosmetic composition and method for the protection of skin and keratin fibres from the harmful effects of radiation, particularly from the harmful effects caused by radiation of blue light or high-energy visible (HEV) light. In particular, the method for the protection of skin and keratin fibres from the effects of blue light comprises topically applying a cosmetic composition comprising lingonberry extract or lingonberry powder on the skin or keratin fibres.

EP 3 964 194 A1

**Description**

FIELD

The present invention relates to a cosmetic composition and method for the protection of skin and keratin fibres from the harmful effects of radiation, particularly from the harmful effects caused by radiation of blue light or high-energy visible (HEV) light. In particular, the method for the protection of skin and keratin fibres from the effects of blue light comprises topically applying a cosmetic composition comprising lingonberry extract or lingonberry powder on the skin or keratin fibres.

BACKGROUND

High-energy visible (HEV) light or blue light is a high-frequency, short-wave light in the violet/blue band with wavelengths that typically range from 380 to 530 nanometers. The majority of our exposure to blue light is caused by the sun, but blue light is also emitted by devices like smartphones, computer screens, tablets and televisions along with fluorescent and LED bulbs of indoor lightning. Although the energy emitted by the above-mentioned electronic devices may be weak, their daily use and location of users very close to the light source increases exposure to blue light.

Long-term blue light exposure to concentrated sources of blue light energy can cause skin damage, including color changes and weakening of the skin's surface. A study by Nakashima et al (2017) showed that blue light contributes to the formation of free radicals and induces oxidative stress in live skin. Blue light thus promotes skin photoaging, meaning aging from exposure to light, similar to UVA. Visible light may thus cause a lot of damage, including formation of free radicals, oxidative DNA damage, suppression of innate immunity and premature photoaging.

Moreover, many studies have reported that blue, green and red light separately exert diametrically opposed effects, for instance on fibroplast proliferation. Fibroblasts generally grow faster after exposure to red or green light, while blue light has antiproliferative effects (Rascalou et al, 2018).

According to Hettwer et al (2017) blue light is an underestimated threat leading to elevated ROS (reactive oxygen species) in epithelial tissue. Main impact is the generation of intracellular ROS as a consequence of shifting blue light absorbing molecules (photosensitizers) into an excited state. According to Hettwer, antioxidative molecules most likely support the cells to reduce the ROS generated by blue light irradiation or suppress the basal level of ROS, which are generally appearing intracellularly. Thus, supplementation of the skin with antioxidants might reduce the ROS content in the skin.

In patent literature various patent applications disclose methods and compositions for protecting human skin from the effects of blue light. For instance, patent application WO 2016176845 A1 describes antioxidant compositions for protecting skin, hair and nails against exposure to high energy visible light. The compositions include one or more natural oils or extracts, such as seabuckthorn berry oil, chaga extract, cumin oil, pumpkin oil, turmeric oil, hemp seed oil, wheat germ oil, rosehip oil, cranberry oil, and broccoli seed oil.

US patent application publication US 20130078205 A1 discloses compositions for the protection of skin from HEV radiation. The compositions contain a melamin derivative, optionally together with plant extracts and natural oils, such as rice bran oil. KR102087868A suggests a combined extract of *Stellaria media* (chickweed), *Helianthus annuus* (sunflower) and cowberry as a cosmetic ingredient for protecting skin form ultraviolet rays and blue light.

JP patent publication JP 2015044773 A suggests compositions comprising bilberry extract for suppressing the cell growth inhibition caused by radiation of blue light. WO 2006134583 A1 relates to use of an aqueous extract of a combination of lingonberry fruit and leaf extract in the manufacture of cosmetic compositions for treating skin conditions.

However, due the underestimated threat caused by increasing exposure of humans to blue light further attempts, methods and compositions for protecting human skin and keratin fibres from harmful effects of blue light or high energy light are needed.

SUMMARY OF THE INVENTION

The invention is defined by the features of the independent claims. Some specific embodiments are defined in the dependent claims.

The present invention is based on the finding that ingredients derived from lingonberry, in particular lingonberry extracts and spray-dried lingonberry powder, have a protective effect against blue light irradiation-induced ROS production by keratinocytes. In particular, even a concentration of lingonberry extract below 0.01% presented a strong protective effect against blue light irradiation-induced ROS production (42% of protection), when tested with blue light wavelengths of 380-530 nm.

According to a first aspect of the present invention, there is thus provided a method of protecting human skin and keratin fibres from effects of high energy visible light comprising a wavelength of about 380 nm to about 530 nm, the method comprising topically applying on the skin or keratin fibres a cosmetic composition comprising lingonberry extract or lingonberry powder or both as an effective agent in an amount of 0.001% to about 50% by weight of the composition, together with a cosmetically acceptable carrier.

According to a second aspect of the present invention, there is provided a cosmetic composition for use in protecting human skin and keratin fibres from effects of high energy visible light, comprising lingonberry extract, lingonberry powder or both in an amount of 0.001% to about 50% by weight of the composition, together with a cosmetically acceptable carrier, for topical application of the composition to skin or keratin fibres.

Considerable advantages are obtained by the invention. First, the composition and method of the present invention reduce the risk of damage to skin and keratin fibres from the effects of HEV radiation and help maintain the appearance and condition of the skin and keratin fibres. The present compositions and methods also reduce the risk of transepidermal water loss (TEWL, drying of skin), skin pigmentation disorders as well as the risk of premature aging of the skin, such as wrinkles and other visual indications of skin aging, like loss of skin tone and elasticity.

In particular, the compositions and methods of the present invention provide a strong protective effect against blue light irradiation-induced ROS production by keratinocytes. The cosmetic compositions of the invention comprising lingonberry extract or lingonberry powder or both effectively block blue light irradiation, when tested with blue light wavelengths of 380-530 nm.

Further features and advantages of the present technology will appear from the following description of some embodiments.

BRIEF DESCRIPTION OF THE DRAWINGS

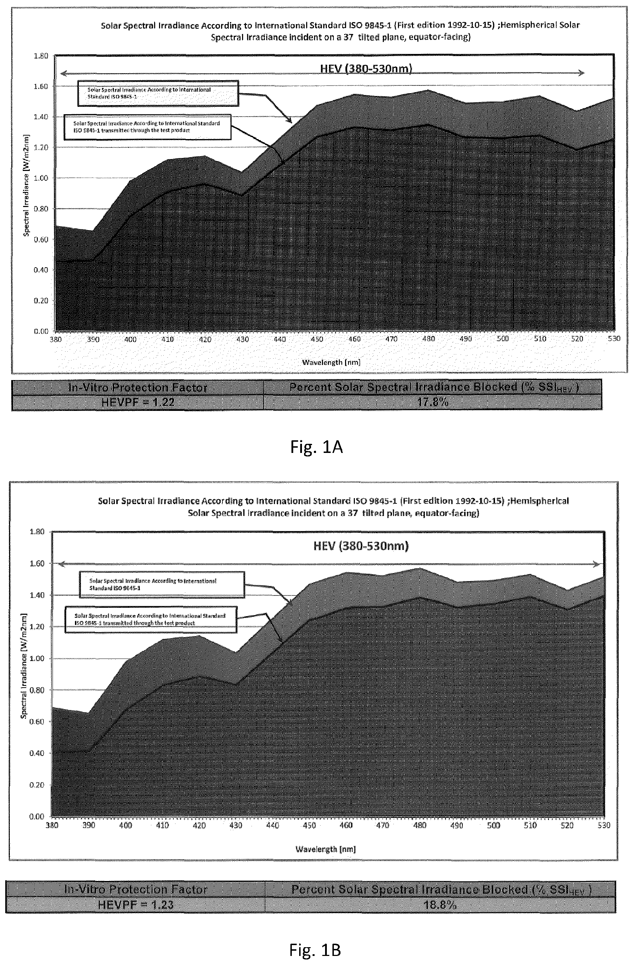

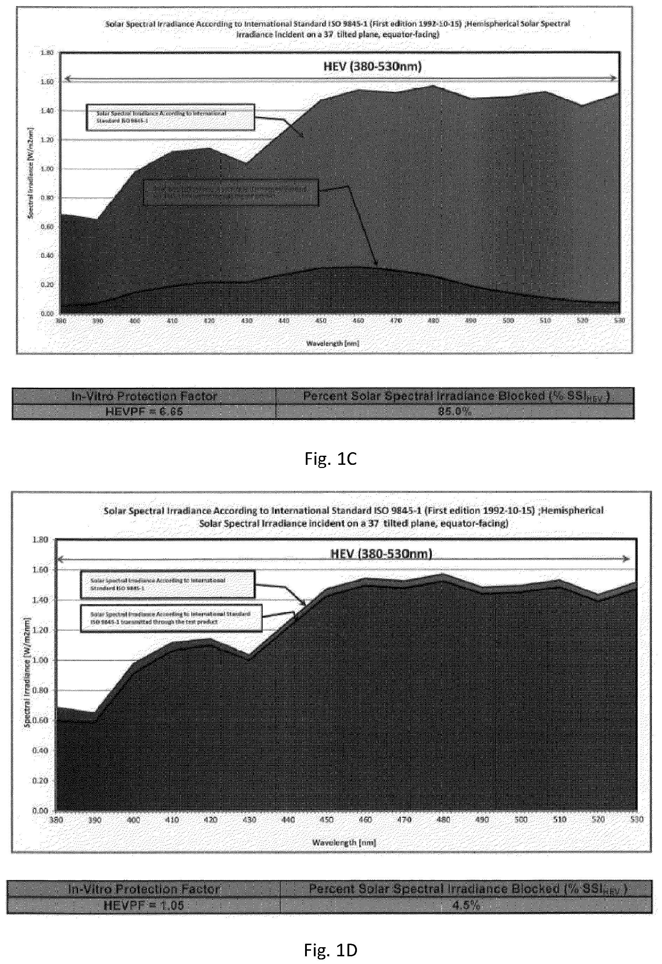

FIGURES 1A-D illustrate the total radiation vs. blocked irradiation at certain wavelengths using lingonberry extract fraction F1 (FIG. 1A); lingonberry extract fraction F3 (FIG. 1B); 100% spray-dried lingonberry powder (FIG. 1C); and 45% spray-dried lingonberry powder (FIG. ID).

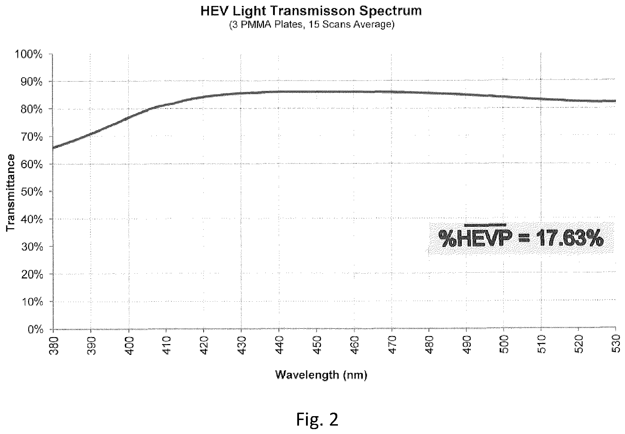

FIGURE 2 illustrates the in vitro protection factor against HEV (HEV-PF) of lingonberry extract fraction F1.

FIGURE 3 illustrates example process steps of preparing lingonberry extract fractions, in particular lingonberry extract fractions F1 and F3, according to EP 2914242 B1.

EMBODIMENTS

DEFINITIONS

In the present context, the term "high energy visible light" or "blue light" comprises wavelengths of about 380 nm to about 530 nm of the visible light region, in particular wavelengths of about 400 to 500 nm. In general, wavelengths of visible light are within about 400-760 nm, while IR (infrared radiation) comprises wavelengths of about 740 to 1000 nm.

Within this disclosure, "lingonberry extract" refers to lingonberry extracts, which typically have a total quercetin concentration of at least 1 mg/g dry matter and/or a total phenolic content of at least 20 mg/g dry matter. In some embodiments, "lingonberry extract" refers to lingonberry extract fractions obtained according to the method disclosed in EP 2914242 B1, particularly to lingonberry extract fractions F1 and F3 obtained according to the method disclosed in EP 2914242 B1.

In the present context, "lingonberry powder" refers to spray-dried lingonberry powder, which typically has a total quercetin concentration of at least 0.4 mg/g dry matter and/or a total phenolic content of at least 10 mg/g dry matter. Spray-dried lingonberry powder is typically obtained by pressing the juice from lingonberry fruit (berries), filtering and mixing the juice with maltodextrin, and then spray-drying the mixture (spray-dried juice powder). However, spray-dried lingonberry powder may also be obtained by spray-drying lingonberry fruit extracts, such as extracts of lingonberry press cake, which typically have been further processed or enriched e.g. by chromatography to obtain higher concentrations of total quercetin and total phenolic compounds in the lingonberry powder. Within this context, "lingonberry powder" refers to lingonberry powders obtained by either of the above disclosed methods. In some embodiments, "spray-dried lingonberry extract powder" or "spray-dried extract powder" may refer particularly to a spray-dried enriched extract.

Within this disclosure, "lingonberry" refers to fruit, *i.e.* berries of lingonberry *(Vaccinium vitis-idaea)*. Other parts of lingonberry, such as twigs, roots and leaves of lingonberry are excluded.

A "total quercetin concentration" or "an amount of quercetin" refers to a combined concentration or combined amount of free quercetin and quercetin in glycoside form. Quercetin analysis was performed by a liquid chromatography method as disclosed in EP 291424 B1.

As stated above, it has been found that lingonberry extracts and lingonberry powder have a protective effect against blue light irradiation-induced ROS production by keratinocytes. In particular, even a concentration of lingonberry extract below 0.01% presented a strong protective effect against blue light irradiation-induced ROS production (42% of protection), when tested with blue light wavelengths of 380-530 nm.

Lingonberries *(Vacciniun vitis-idaea)* are known to contain e.g. quercetin and kaempferol glycosides among other phenolic compounds. The phenolic composition of lingonberries has been widely studied but it is not yet characterized thoroughly. The amounts of different compounds are known to vary according to many factors, such as the growing environment. The conjugated structures also cause additional challenge to analyzing. The main classes of phenolic compounds found in lingonberries are hydroxybenzoic acids, hydroxycinnamic acids, anthocyanins, flavonols, flavan-3-ols and proanthocyanidins.

Quercetin has numerous advantageous characteristics, e.g. antioxidative and anti-inflammatory properties. Quercetin is categorized as a flavonol by structure. All flavonols have in common a 3-hydroxyflavone backbone, which can carry further phenolic OH groups in various positions. These compounds as well as phenolic compounds in general are present in many fruits, berries and vegetables and they mostly appear as conjugated glycosides, i.e. bound to various sugar molecules.

Exploiting for example the press cake of lingonberry and other berries as a source of phenolic compounds is as such known in the art. The commercial interest has been mainly focused on obtaining extracts rich in anthocyanins and proanthocyanidins.

A preferred option to make quercetin-rich extracts of lingonberry origin for cosmetic application is disclosed in EP 2914242 B1, which is incorporated herein by reference.

In the present invention it was found that fractions of lingonberry, especially lingonberry fractions rich in phenolic compounds, anthocyanins and quercetin, in particular fractions rich in total phenolic compounds, protect human skin from effects of blue light or high energy light, typically HEV having a wavelength of about 380-530 nm.

In particular, fractions of lingonberry extract prepared according to EP 2914242 B1, especially fractions F1 and F3, as well as spray-dried lingonberry powder and enriched lingonberry extracts/spray-dried enriched extracts prepared by an alternative extraction process compared to that of EP2914242 B1 were found to protect human skin from effects of blue light or high energy light, typically HEV having a wavelength of about 380-530 nm.

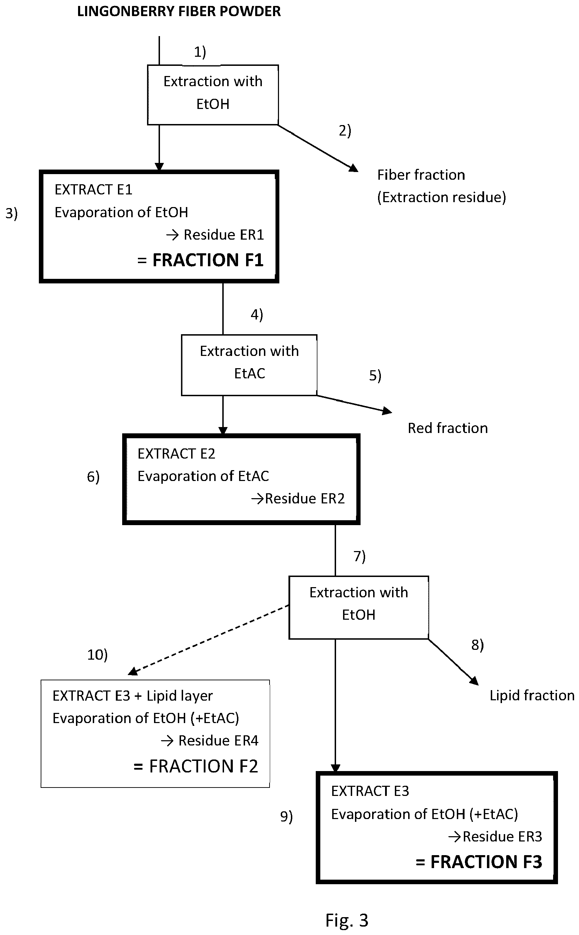

In the method according to EP 2914242 B1 the lingonberry extracts are obtained from lingonberry press cake, specifically from its dried fibrous part (lingonberry fiber powder). The fractionation method is based on three extractions in sequence (Figure 3), the extraction steps being carried out successively with ethanol, ethyl acetate and ethanol. Consequently, lingonberry fiber powder is first extracted by ethanol (EtOH). After evaporation the residue may be separated to two fractions by the second extraction with ethyl acetate (EtAC). After a few intermediate steps, another ethanol extraction yields two additional fractions. All of the finished fractions include very low amounts of the solvents used. Thus in one embodiment the lingonberry extract has been obtained by stepwise extraction of lingonberry fiber powder with one or more solvents, preferably solvents selected from ethanol and ethyl acetate.

After lingonberry extract is fractionated as disclosed above, the concentrations of total phenolic compounds, as well as the quercetin concentration, and antioxidant capacities (ORAC) of the fractions of main interest are high. In particular, this applies to fractions F1 and F3 obtained by the above-disclosed method.

Alternatively, lingonberry extracts may be obtained for example from lingonberry press cake, wherein the method comprises solvent extraction, typically ethanol extraction, followed by adsorption chromatography, where phenolic compounds are recovered and enriched while compounds such as sugars, amino acids and salts are removed from the extract. The obtained enriched extract may comprise ≥25% polyphenolic compounds. Said extract may be used as such or preferably spray-dried to obtain lingonberry powder for use in the method and compositions of the invention. For the purposes of the invention, such powders are named as "spray-dried lingonberry powder 100%".

Thus in one embodiment the lingonberry powder has been obtained by a method wherein lingonberry press cake is extracted with an aqueous alcoholic solvent, such as aqueous ethanol, followed by adsorption chromatography of the extract to obtain an enriched extract, which may be spray-dried to obtain spray-dried extract powder. In some embodiments, the enriched extract may be mixed with maltodextrin before spray-drying.

The above disclosed two methods for obtaining lingonberry extracts or spray-dried lingonberry extracts start from lingonberry press cake or its dried form (lingonberry fiber powder). However, it is also possible to use lingonberry juice, which is pressed from lingonberry fruit (berries) as a starting material when preparing lingonberry fractions, which have the same advantageous effects as the above disclosed extracts obtained from lingonberry press cake.

Thus it was found that also spray-dried lingonberry powder obtained by spray-drying lingonberry juice protects the keratinocytes of human skin against harmful effect of blue light or high energy light. The spray-dried lingonberry powder for use in the method and composition of the invention may thus be prepared simply by pressing the juice from lingonberry fruit, filtering and mixing the juice with maltodextrin, and then spray-drying the mixture. Preferred ratios are for example 30% of lingonberry juice and 70% of maltodextrin or more preferably 45% of lingonberry juice and 55% of maltodextrin, wherein the percentages are given per weight. For the purposes of the invention, such powders are named as "spray-dried lingonberry powder 30%" or "spray-dried lingonberry powder 45%". The powder so obtained may be used in the cosmetic compositions of the present invention. The concentration of the powder in a typical composition is 0.001 - 50 %, more preferably 0.001 - 20%, of the total weight of the composition.

Thus in one embodiment the lingonberry powder has been obtained by a method wherein lingonberry juice is pressed from lingonberry fruit, filtered and mixed with maltodextrin, and the mixture is spray-dried.

In one embodiment, the cosmetic composition contains lingonberry extract or lingonberry powder or both in an amount of 0.001 to 20%, preferably from 0.007% to about 20%, more preferably in an amount of 0.01 to 20%, by weight of the composition.

In a preferred embodiment the cosmetic composition contains lingonberry extract or lingonberry powder in an amount of 0.007 — 20 %, preferably 0.01 - 20 % by weight.

In a preferred embodiment, the lingonberry extract or lingonberry powder comprises quercetin in an amount of at least 0.4 mg/g dry matter, such as 0.4-30 mg/g dry matter, preferably 1-30 mg/g dry matter. The amount of polyphenolic compounds in the lingonberry extract or lingonberry powder is typically 2—400 mg/g dry matter, in particular at least 10 mg/g dry matter, such as 10—400 mg/g dry matter, preferably at least 20 mg/g dry matter, such as 20-400 mg/g dry matter.

Antioxidant capacities (ORAC) of the above disclosed lingonberry extracts and lingonberry powders usually correlate with the quercetin concentrations and the concentrations of the phenolic compounds. Typically, lingonberry extracts have ORAC values of 200 — 1000 μmol TE/g but the ORAC values of enriched lingonberry extracts may reach a level of 6000—7000 μmol TE/g. However, also spray-dried lingonberry powder without enriching shows ORAC values of 200 — 1000 μmol TE/g, although its quercetin and phenolic concentrations are somewhat lower than those of lingonberry extracts.

The composition of the invention is supposed to be used topically. The formulations of the composition include but are not restricted to compositions selected from the group consisting of skin care cream, skin care lotion, skin care gel, skin softener, skin toner, ointment, milk lotion, moisture lotion, nutrition lotion, nutrition cream, emulsion cream, moisture cream, hand cream, skin serum, essence, nutrition essence, zinc oxide based cream (with sun protection factor), cosmetic tonic water, cleansing foam, cleansing lotion, cleansing cream, cleansing gel, body lotion, body cleanser, makeup base, foundation cream, pressed powder, loose powder, concealer, primer, mascara, lipstick, lip gloss, lip cream, lip gel, eye shadow, make-up setting spray, leave-on treatment for scalp and hair, and hair styling products.

In an embodiment, a cosmetic composition according to the invention contains, depending on the intended use of the composition, at least one of lingonberry fraction F1, lingonberry fraction F3 and lingonberry powder in an amount of 0.001 to 50 % by weight, more preferably the amount of the fractions or powder is 0.001 to 20 % by weight, 0.007 to 20% by weight or 0.01 to 5 % by weight, and most preferably 0.01 to 1 % by weight.

In an embodiment, the cosmetic composition comprises at least two of lingonberry fraction F1, lingonberry fraction F3 and lingonberry powder. In a further embodiment, the cosmetic composition according to the invention comprises lingonberry fraction F1, lingonberry fraction F3 and lingonberry powder.

In a further embodiment, the cosmetic composition comprises the enriched form of lingonberry powder (i.e. spray-dried enriched lingonberry extract) as the effective agent or as the sole effective agent protecting skin and keratin fibres from the harmful effects of blue light or HEV light.

The effect of protecting skin and keratin fibres from the harmful effects of blue light or HEV light may be provided solely by lingonberry extract, lingonberry powder or both as effective agents. However, the cosmetic composition according to the invention may also contain other extracts or oils from Nordic plants as discussed below.

In embodiments, the cosmetic composition according to the invention may also contain, depending on the intended use of the composition, other extracts or oils from Nordic plants, in addition to lingonberry extract and/or lingonberry powder. Typically, other extracts or oils from Nordic plants are present in an amount of 0.1 to 25 % by weight, more preferably in an amount of 0.1 to 5 % by weight, and most preferably 0.5 to 3 % by weight.

Nordic plants include flowers, trees, mushrooms, grains, herbs, algae and berries from the Nordic region. Nordic plants thus include but are not limited to birch (*Betula*), spruce (*Abies*), pine (*Pinus*), heather (*Calluna vulgaris*), meadowsweet (*Filipendula ulmaria*), Nordic cottongrass (*Eriophorum spissum*), marygold (*Calendula officinalis*), willowherb (*Epilobium*), chaga (*Inonotus obliquus*), sheep polypore (*Albatrellus ovinus*), red algae (*Furcellaria*), hemp (*Cannabis sativa*), oat (*Avena sativa*), canola/rapeseed (*Brassica*), as well as other Nordic berries in addition to lingonberry, typically including but not limited to blueberry (*Vaccinium myrtillus*), cloudberry (*Rubus chamaemorus*), cranberry (*Vaccinium oxycoccus* / *Oxycoccus palustris*), small cranberry (*Vaccinium microcarpum*), bog bilberry (*Vaccinium uliginosum*), raspberry (*Rubus idaeus*), crowberry (*Empetrum nigrum*), rowanberry (*Sorbus aucuparia, Sorbus fennica*), sea buckthorn (*Hippophaë rhamnoides*), blackcurrant (*Ribes nigrum*), redcurrant (*Ribes rubrum*), white currant (*Ribes rubrum f. leucocarpum*), greencurrant (*Ribes nigrum*), gooseberry (*Ribes uva-crispa*), wild recurrant (*Ribes spicatum*), mountain currant (*Ribes alpinum*), aronia berry (*Aronia mitschurinii*), saskatoon (*Amelanchier alnifolia*), Arctic bramble (*Rubus arcticus*), stone bramble (*Rubus saxatilis*), strawberry (*Fragaria x ananassa*), blackberry (*Rubus fruticosus*), bearberry (*Arctostaphylos uva-ursi*), alpine bearberry (*Arctostaphylos alpine*), wild strawberry (*Fragaria vesca*), juniper berry (*Juniperus communis*), wild rose (*Rosa acicularis*), dog rose (*Rosa canina*), cinnamon rose (*Rosa cinnamomea* / *Rosa*

*majalis*), glaucous dog rose (*Rosa dumalis*), Rubus species *Rubus pruinosus*, blackthorn (*Prunus spinosa*), Cornelian cherry (*Cornus mas*), bunchberry (*Cornus suecica*), dewberry (*Rubus caesius*), Finnish whitebeam (*Hedlundia hybrida*) and bird cherry (*Prunus padus*).

In one embodiment, the other oils and/or extracts are selected from chaga extract, cloudberry seed oil and seabuckthorn berry oil, which may further enhance the effect of protecting skin from harmful effects of blue light.

The skin care cream composition according to the invention can be of the type oil-in-water emulsion, water-in-oil emulsion, water-oil-water emulsion or a microemulsion. The emulsion cream composition according to the invention contains at least one of the lingonberry fractions F1 and F3 and lingonberry powder as defined herein in an amount of preferably 0.001 to 50 % by weight, more preferably 0.001 to 20% by weight or 0.01 to 5 % by weight and most preferably 0.01 to 1 % by weight. The skin care composition may also be a lotion, or a gel. The content of other extracts or oils from Nordic plants in a skin care composition may be in an amount of 0.1 to 25 % by weight. Preferably the amount of other plant oils or extracts is 0.1 to 5 % by weight and most preferably 0.5 to 3 % by weight.

The cosmetically acceptable carrier in the compositions of the invention comprises a liquid, a powder, or a wax, or more than one of these.

The skin care compositions according to the invention may contain one or more adjuvants acceptable in the field of cosmetics, such as preservation agents, thickening agents, moisturizing agents, and other suitable additives, such as for example perfumes and/or colouring agents. Suitable preservation agents are for example various organic acids and phenoxyethanol, in addition to ingredients that are not preservatives according to Cosmetic Regulation but inhibit microbial growth, like organic acids, alcohols, like phenethyl alcohol and benzyl alcohol, glycols, ethylhexylglycerin, phenyl propanol, glyceryl caprate and sodium levulinate. These preservation agents can be used alone or combined with each other. As thickening agent any thickening agent suitable in the field of cosmetics can be used, as long as it is compatible with the other components of the composition, for example xanthan gum, carbomer, hydroxyethylcellulose, hydroxypropylmethylcellulose, cellulose gum, Sclerotium gum, Acacia Senegal Gum, Chondrus Crispus, cetearyl dimethicone crosspolymer and magnesium aluminium silicate. Thickening agents can be used alone or in combination with each other. Suitable moisturizing agents are for example glycerin, propylene glycol, butylene glycol, pentylene glycol, heptyl undecylenate, hyaluronic acid, betaine, xylitol and its derivatives, trehalose, or sodium PCA. In the emulsion creams according to the invention, also different skin conditioning agents, like for example vitamins, antioxidants, tocopheryl acetate, and ethylhexylglycerin may be used.

In addition, one or more compounds acting as an emulsifier, *i.e.* a compound dispersing and stabilizing the oil in water is needed in the emulsion cream composition. Useful emulsifiers are all non-ionic emulsifiers accepted by the cosmetic legislation, such as, for example, glyceryl stearate, PEG stearate, ceteareth, sucrose stearate/distearate, sodium stearoyl glutamate, sorbitan olivate, glyceryl stearate citrate, PEG-5 glyceryl stearate, PEG-100 stearate, PEG-30 dipolyhydroxystearate, lecithin, hydrogenated lecithine and PEG-8 bees wax, steareth-21, steareth-2 as well as a mixture of a fatty glucoside, such as for example cetearyl, cocoyl, cetearyl glucoside, or myristyl glucoside and a fatty alcohol, such as for example cetearyl, cetyl, stearyl, octyldodecanol, caprylic/capric triglyceride or myristyl alcohol. In addition, of anionic emulsifiers, for example stearic acid, sodium hydroxide, triethanolamine and aminomethyl propanediol are useful. Also different chelating agents, like disodium EDTA, sodium phytate, sodium gluconate and citric acid may be used.

In an embodiment, the cosmetic composition may thus comprise at least one of UVA/UVB filters, oils, waxes, fats, moisturizing agents, thickeners, vitamins, antioxidants, bulking agents, emulsifying agents, preserving agents, coloring agents, perfumes, film forming agents, foam boosting agents, emollients, and aqua.

One embodiment of the invention is a zinc oxide based cream or ointment, which contains at least one of the lingonberry fractions F1 and F3 and lingonberry powder as defined herein in an amount of preferably 0.001 to 20 % by weight, more preferably 0.01 to 5 % by weight and most preferably 0.01 to 1 % by weight. The zinc oxide based cream contains an amount of zinc oxide, which is 0.01 to 25% by weight, preferably 0.1 to 20% by weight, and more preferably 0.5 to 10% by weight. The content of other extracts or oils from Nordic plants in a skin care composition may be in an amount of 0.1 to 25 % by weight. Preferably the amount of other plant oils or extracts is 0.1 to 5 % by weight and most preferably 0.5 to 3 % by weight.

A further embodiment of the invention is a tonic cosmetic water, which contains at least one of the lingonberry fractions F1 and F3 and lingonberry powder as defined herein in an amount of preferably 0.001 to 20 % by weight, more preferably 0.01 to 5 % by weight and most preferably 0.01 to 1 % by weight. A typical tonic water according to the invention contains ethanol in an amount which is 1 to 70 % by weight, preferably 5 to 50% by weight, together with suitable moisturizing agents, oils, emulsifying agents, thickening agents, and antimicrobial agents. Suitable alternatives of said agents include those mentioned above in connection of skin care compositions. Further, the tonic water may also comprises oils and extracts from other Nordic plants, for example in an amount of 0.1 to 25 % by weight. Preferably the amount of other plant oils or extracts is 0.1 to 5 % by weight and most preferably 0.5 to 3 % by weight.

The lipstick compositions according to the invention contain at least one of the lingonberry fractions F1 and F3 and lingonberry powder as defined herein in an amount of preferably 0.001 to 20 % by weight, more preferably 0.01 to 5 % by weight and most preferably 0.01 to 1 % by weight. In addition, these compositions may contain extracts and oils from other Nordic plants in an amount of 0.01 to 10 % by weight. Preferably the amount of other plant oils or extracts is 0.1 to 5 % by weight and most preferably 0.5 to 3 % by weight

The lipstick compositions according to the invention contain in addition to at least one of the lingonberry fractions F1 and F3 and lingonberry powder as defined herein also other substances, like different waxes, naturally derived oils, coloring and pearlescent agents, which are acceptable in the field of cosmetics and which are traditional components of lipstick compositions. Usable waxes are the natural waxes, such as bees wax, candelilla, carnauba, cereal based waxes, jojoba wax and their derivatives, berry fruit wax and sunflower seed wax, in addition to different type of stability enhancers like silica and magnesium stearate. Usable oils are the natural and naturally derived oils, such as oat oil, caprylic/capric triglycerides and octyldodecanol, including oils from Nordic berries, e.g. lingonberry seed oil, cranberry seed oil and bilberry seed oil. Lipstick compositions according to the invention can also contain emulsifiers and dispersing aids like PEG-8, behenyl alcohol and arachidyl alcohol, polyglyceryl-2 triisostearate and various vitamins and derivates of those, like tocopherol and ascorbyl palmitate.

The lipstick compositions according to the invention can in addition contain one or more adjuvants and/or additives acceptable in the field of cosmetics, such as preservation agents. Suitable preservation agents are for example phenoxyethanol and glyceryl caprylate. These preservation agents can be used alone or in combination with each other.

The cleansing compositions according to the invention contain at least one of the lingonberry fractions F1 and F3 and lingonberry powder as defined herein in an amount of preferably 0.001 to 20 % by weight, more preferably 0.01 to 5 % by weight and most preferably 0.01 to 1 % by weight. The cleansing compositions may further contain other extracts or oils from Nordic plants in an amount of 0.01 to 25 % by weight. Preferably the content of other plant oils or extracts is 0.1 to 5 % by weight and most preferably 0.5 to 3 % by weight.

The cleansing compositions according to the invention typically contain also one or more other substances acceptable in the field of cosmetics such as emulsifying agents, solvents, preservatives, oils, waxes and fats, moisturizing agents or humectants, foam boosting agents, thickeners and colorants. These substances may be e.g. sodium lauroyl sarcosinate, sodium laureth sulfate, glycerin, propylene glycol, betaine derivatives, glucose derivatives, hydroxypropyl methylcellulose, cocamide MEA, xanthan gum, carrageenan, PEG-200 glyceryl stearate and PEG-120 methyl glucose trioleate.

The foundation cream/emulsion composition according to the invention contains at least one of the lingonberry fractions F1 and F3 and lingonberry powder as defined herein in an amount of preferably 0.001 to 20 % by weight, more preferably 0.01 to 5 % by weight and most preferably 0.01 to 1 % by weight. The content of other extracts or oils from Nordic plants in a foundation composition may be in an amount of 0.1 to 25 % by weight. Preferably the amount of other plant oils or extracts is 0.1 to 5 % by weight and most preferably 0.5 to 3 % by weight.

The foundation cream/emulsion compositions according to the invention contain, in addition, one or more adjuvants acceptable in the field of cosmetics, such as preservation agents, thickening agents, moisturizing agents, UV filters and other suitable additives, such as for example perfumes and/or coloring agents. Suitable preservation agents are for example various organic acids and phenoxyethanol, in addition to ingredients that are not preservatives according to Cosmetic Regulation but inhibit microbial growth, like organic acids, alcohols, like phenethyl alcohol and benzyl alcohol, glycols, ethylhexylglycerin, phenyl propanol, glyceryl caprate and sodium levulinate. These preservation agents can be used alone or combined with each other. As thickening agent any thickening agent suitable in the field of cosmetics can be used, as long as it is compatible with the other components of the composition, for example hectorites, glyceryl stearate, xanthan gum, carbomer, hydroxyethylcellulose, hydroxypropylmethylcellulose, Sclerotium gum, Chondrus Crispus, acrylates and acrylate derivatives, cetearyl dimethicone crosspolymer and magnesium stearate and magnesium aluminium silicate. Thickening agents can be used alone or in combination with each other. Suitable moisturizing agents are for example glycerin, propylene glycol, butylene glycol, heptyl undecylenate, hyaluronic acid, betaine, xylitol or sodium PCA. As UV filters, components of natural origin, like zinc oxide and titanium dioxide are preferred. UV filters can be used alone or in combination with each other. In the emulsion creams according to the invention, also different skin conditioning agents, like for example vitamins, antioxidants, tocopheryl acetate, and ethylhexylglycerin, as well as known emulsifying agents, bulking agents and silicones and volatile silicon alternatives like ethyl oleate, C15-19 alkane, and isododecane and its natural origin alternatives like C13-15 alkanes, may be used.

Examples of keratin fibres include but are not limited to human hair, eye lashes, eye brows, beard and moustache (facial hair), in particular eye lashes. Compositions according to the invention for topical application on keratin fibres include but are not limited to hair coloring agents, shampoos, conditioners, mascaras, eye brow coloring products, and beard and moustache cleansing and conditioning products.

The mascara compositions according to the invention contain at least one of the lingonberry fractions F1 and F3 and lingonberry powder as defined herein in an amount of preferably 0.001 to 25 % by weight, more preferably 0.01 to 5 % by weight and most preferably 0.01 to 1 % by weight. The mascara compositions may further contain other extracts or oils from Nordic plants in an amount of 0.01 to 25 % by weight. Preferably the content of other plant oils or extracts is 0.1 to 5 % by weight and most preferably 0.5 to 3 % by weight.

The mascara compositions according to the invention typically contain also other substances acceptable in the field of cosmetics such as oils, waxes and fats, film forming polymers, colorants, color additives, emulsifying agents, moisturizing agents, preservatives and antioxidants. Suitable emulsifying agents are e.g. stearates, stearic acid and its neutralizing agents. As thickening agent any thickening agent suitable in the field of cosmetics can be used, as long as it is compatible with the other components of the composition, for example hectorites, glyceryl stearate, xanthan gum, carbomer, hydroxyethylcellulose, hydroxypropylmethylcellulose, Sclerotium gum, Acacia Senegal gum, Chondrus Crispus, magnesium stearate and magnesium aluminium silicate. Thickening agents can be used alone or in combination with each other. Usable waxes are the natural waxes, such as bees wax, candelilla, carnauba, cereal based waxes, jojoba wax and their derivatives, berry fruit wax and sunflower seed wax, in addition to different type of stability and rheology modifiers and bulking agents like silica.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

EXPERIMENTAL

**Example 1.** Preparation of fractions F1 and F3 from lingonberry fiber powder

400 g of lingonberry fiber powder (Kiantama Oy, Suomussalmi, Finland) was extracted with four litres of ethanol (ETAX A, Altia Oyj, Rajamäki, Finland) in ultrasonic bath (Grant Ultrasonic bath XB22, Grant Instruments (Cambridge) Ltd, Shepreth, England, 38 kHz,) for 3 × 20 min, stirring the mixture between ultra sound treatments. The fibrous extraction residue was separated by filter paper (5 μm) in Büchner funnel (Vacuum pump, MZ 2C+2AK, Vacuubrand GMBH+CO KG, Wertheim, Germany) to obtain extract **E1** (Figure 3).

Ethanol was evaporated from E1 in Rotavapor (R120 prof V, Büchi Labortechnic AG, Zurich, Switzerland) at 45°C, min 70 mbar. Ca. 90 g of dark red viscous paste-like evaporation residue ER1 was obtained. It was designated as Fraction **F1** ("Lingonberry Fraction F1") ready to be used in cosmetic compositions as such.

Fraction F1 was then extracted with 400 ml of ethyl acetate (EMSURE®, Merck KGA, Darmstadt, Germany). The red fraction was separated by filter paper (5 μm) in Büchner funnel to obtain Extract **E2**. The ethyl acetate was evaporated from E2 in Rotavapor (45°C, min 70 mbar), whereby the evaporation residue **ER2** was obtained.

ER2 was extracted with 300 ml of ethanol. The lipid fraction was separated by decanting to obtain Extract **E3**. When ethanol was evaporated from E3 in Rotavapor (45°C, min 70 mbar) ca. 18 g of orange red evaporation residue **ER3** was obtained. It was designated as Fraction F3 ("Lingonberry Fraction F3"), ready to be used in cosmetic compositions as such.

Quercetin and total phenolic content in addition to oxygen radical absorbance capacity (ORAC) were measured from the fractions F1 and F3 as disclosed in EP 2914242. Briefly, quercetin analysis was thus performed by liquid chromatography method (HPLC), wherein the analysis instrument was equipped with a photodiode array detector (DAD). Oxygen radical absorbing capacity (ORAC) analysis was performed by the method of Huang *et al.,* 2002, where the results were calculated as trolox equivalents (TE).

Fractions F1 and F3 obtained as disclosed above had quercetin concentrations of 2.4 mg/g and 4.2 mg/g, respectively. The highest quercetin concentration, 4.2 mg/g, in fraction F3 is about ten-fold higher than in the original lingonberry fiber powder (0.4 mg/g). Total phenolic content, analysed by Folin-Ciocalteu method (Magalhães *et al.,* 2010), was 21 mg/g for fraction F1 and 28 mg/g for fraction F3. Antioxidant capacities (ORAC) of fractions F1 and F3 were 290 $\mu$mol TE/g and 750 $\mu$mol TE/g, respectively.

Analysis from industrial scale production of fractions F1 and F3 are summarized in Table 1 below. The results are averages from several lingonberry batches harvested and further processed during various years. There is natural variation among the concentrations of various batches of lingonberries due to different growing seasons, harvesting sites etc.

Further, fractions F1 and F3 were tested for their ability to inhibit the formation of ROS under exposure to blue light and block solar spectral irradiance, as indicated in Examples 3 and 4.

**Example 2.** Preparation of spray-dried lingonberry powder from lingonberry juice

Lingonberries (*Vacciniun vitis-idaea*) were collected from a forest in northern Finland. The berries were warmed and treated with pectinase, and the juice was pressed using a conventional presser. Subsequently, the juice was filtered, pasteurized and concentrated. The concentrate was diluted with water and mixed with maltodextrin in a ratio of 30% of lingonberry juice and 70% of maltodextrin (per weight) and the mixture was spray-dried in a conventional spray-drier, by leading the concentrated mixture into the drying chamber as spray, whereby water was evaporated and the dried particles separated. The powder was cooled down, sieved out and packed. The powder so obtained was named as "spray-dried lingonberry powder 30%". In a similar manner, "spray-dried lingonberry powder 45%" or "spray-dried lingonberry juice powder 45%" was prepared, with a mixing ratio of 45% of lingonberry juice and 55% of maltodextrin (per weight).

The spray-dried lingonberry powder 45% had a total quercetin content, i.e. the total amount free quercetin and quercetin in glycoside form, of 47 mg/100 g powder. The total amount of phenolic compounds was 1.2 g/100g. The ORAC value, indicating the antioxidant capacity of the spray-dried lingonberry powder was 23681 $\mu$mol TE /100g powder.

The ORAC analysis was performed by the method of Huang *et al,* 2002, where the results were calculated as trolox equivalents (TE). The analysis method for free quercetin was HPLC-DAD. Total quercetin (free + glycosides) was analyzed with HPLC-DAD after hydrolysis. Phenolic compounds were analyzed using Folin-Ciocalteau analysis method.

Spray-dried lingonberry powder 45% was tested for its ability to block solar spectral irradiance over a spectral range of 380-530 nm and to inhibit the formation of ROS under exposure to blue light, as indicated in Examples 3 and 4, respectively.

The table below contains typical amounts of quercetin and phenolic compounds per dry matter in the spray-dried lingonberry powder 45%, spray-dried lingonberry extract powder 100%, and lingonberry extracts F1 and F3, all obtained as disclosed above and below. The table also shows the antioxidant capacity measured as oxygen radical absorbing capacity (ORAC).

**Table 1.** Analysis results of spray-dried lingonberry extract powder 100%, spray-dried lingonberry juice powder 45% and lingonberry extracts F1 and F3

|  | Spray-dried extract powder 100% | Spray-dried juice powder 45% | Lingonberry extract F 1 | Lingonberry extract F3 |
|---|---|---|---|---|
| Quercetin (free), mg/g | 1 | 0.09 | 1 | 1-2 |
| Quercetin (free + glycosides) mg/g | 11 | 0.47 | 1.49 | 1.6 |
| Phenolic compounds, mg/g | 350 | 12 | 31-32 | 20-40 |
| Antioxidant capacity, $\mu$mol TE/g | 6545 | 237 | 320-495 | 214-700 |

**Example 3.** Preparation of spray-dried lingonberry extract powder from an extract of lingonberry press residue

Lingonberry press cake obtained as a residue from pressing of lingonberries as disclosed in Example 2 was extracted with aqueous ethanol. The lingonberry press cake was either obtained directly from pressing of berries or stored frozen and melted before extraction. The obtained extract was forwarded to adsorption chromatography, where, phenolic compounds (including anthocyanins) were recovered and enriched while compounds such as sugars, amino acids and salts were removed from the extract. The enriched extract was spray-dried to obtain a spray-dried lingonberry extract powder which contained ≥25% polyphenolic compounds. For the purposes of the invention said powder was named as "spray-dried extract powder 100%". Analysis results are given in Table 1 above. Spray-dried extract powder 100% was also tested for its ability to inhibit the formation of ROS under exposure to blue light and block solar spectral irradiance, as indicated in Examples 3 and 4.

**Example 4.** Evaluation of the HEV protection factor and solar spectral irradiance%

High energy visible light protection factor (HEV-PF) was calculated from spectrometric measurements performed over a spectral range of 380-530 nm.

Calculated protection factor and solar spectral irradiance blocked (in %) provide information regarding product efficacy when exposed to solar spectral irradiance according to International ISO 9845-1 (First edition 1992-10-15; Hemispherical Solar Spectral Irradiance incident on a 37° tilted plane, equator-facing). For this purpose, HEV-PF and % of Solar Spectral Irradiance blocked (%SSI) were calculated as a function of product's efficacy. The measurements and calculations were performed by AMA Labs and the results are shown in the Figures.

In vitro HEV-PF were calculated from the following equation:

In-vitro High Energy Visible Light Protection Factor (*HEVPF*) calculations:

$$HEVPF = \frac{\int_{\lambda=380nm}^{\lambda=530nm} SUN(\lambda)^* d\lambda}{\int_{\lambda=380nm}^{\lambda=530nm} SUN(\lambda)^* T_{HEV}(\lambda)^C {}^* d\lambda}$$

Where:

$SUN(\lambda)$    -International Standard ISO 9845-1 (380-530nm)
c    - Correction factor (coefficient of adjustment)
$d\lambda$    -Wavelength step (1 nm)

The % solar spectral irradiance (%SSI) blocked by the tested products was calculated using area under the curve analysis with the following equation:

Percent Solar Spectral Irradiance *(%SSI$_{HEV}$)* blocked by the test product (Area Under the Curve Analysis).

$$\%SSI_{HEV} = (1 - \frac{AUC_T}{AUC_U})^*100\%$$

Where:

$\%SSI_{HEV}$    - Percent Solar Spectral Irradiance blocked by the test product
$AUC_u$    -Area Under the Curve of Solar Spectral Irradiance According to International Standard ISO 9845-1
$AUC_T$    - Are Under the Curve of Solar Spectral Irradiance According to International Standard ISO 9845-1 transmitted through the test product

In the first evaluation, lingonberry extract fraction F1 had a %SSI-HEV value of 22.9% and an *in vitro* protection factor (HEV-PF) of 1.30%. For comparison, the corresponding values for pine bark extract were 8.3% (%SSI-HEV) and 1.09% (HEV-PF).

In a full study, lingonberry extract fraction F1 had an *in vitro* protection factor (HEV-PF) of 1.22% and SSIHEV value of 17.8%.

**Example 5.** Effect of lingonberry fractions on ROS production

The irradiation of NHEK (normal human epidermal keratinocytes) with blue light at 3.8 J/cm$^2$ induced a strong production of ROS (a 3.4 fold increase) which was significantly inhibited by the reference compound, vitamin E, tested at 50 $\mu$M (59% of protection). These results were expected and validated the assay.

Under the experimental conditions of the assay, lingonberry extract fraction F3, tested at 0.007%, presented a strong protective effect against blue light irradiation-induced ROS production by keratinocytes (42% of protection). When tested at lower concentrations (0.00078% and 0.0023%), the fraction showed no effect on ROS production in keratinocytes after blue light exposure.

Lingonberry extract fraction F3 was first chosen for the test as it had more phenolic compounds and a higher ORAC value than lingonberry extract fraction F1. It was thus assumed to have a stronger protective effect against ROS. However, it was found that fractions F1 and F3 were almost equal as regards their protective effect against blue light irradiation-induced ROS production.

In the following examples, "lingonberry extract" is listed as an ingredient of the exemplified cosmetic compositions. Although in some cases lingonberry extract may be preferred for the purposes of the invention, spray-dried lingonberry powder and/or lingonberry extract powder may be used instead of or in addition to lingonberry extract. Thus the following examples disclose the use of any one of lingonberry juice powder, lingonberry extract powder and lingonberry extract, or any combination thereof, in the indicated amounts in the exemplified cosmetic compositions.

**Example 6.** Skin care cream, lotion or gel

An example of ingredients for a skin care cream, lotion or gel composition for facial and throat skin care is shown in the following Table 1. The described skin care cream, lotion or gel composition comprises an effective amount of lingonberry extract to protect the skin from effects caused by blue light radiation.

| INGREDIENTS | Max. levels % (w/w) |
|---|---|
| Oils, waxes and fats | 30 |
| Humectants | 20 |
| Thickeners | 12 |
| Ethanol (alcohol denat.) | 10 |
| Lingonberry extract | 0.01-50 |
| Bulking agents | 5 |
| UV filters | 5 |
| Emulsifying agents | 5 |
| Preservatives, antimicrobials | 2 |
| Colorants | 2 |
| Perfume | 1 |
| Aqua | to 100 |

In the above composition, preferred oils include various vegetable oils. Preferred fats include for example long chain alcohols. Humectants include e.g. glycerine and propylene glycol. Examples of thickeners are for example carbomer and xanthan gum. Emulsifying agents include anionic, amphoteric and non-ionic surfactants, such as PEG stearate and ceteareth. Further, also vitamins and antioxidants may be added to the composition.

**Example 7.** Zinc oxide based cream

| INGREDIENTS | Max. levels % (w/w) |
|---|---|
| Oils, waxes and fats | 60 |
| Bulking agents | 30 |
| Zinc oxide | 25 |
| Humectants | 20 |
| Emulsifying agents | 12 |

(continued)

| INGREDIENTS | Max. levels % (w/w) |
| --- | --- |
| Lingonberry extract | 0.01-20 |
| Thickeners | 5 |
| Ethanol (alcohol denat.) | 5 |
| Preservatives, antimicrobials | 1.5 |
| Perfume | 1 |
| Aqua | to 100 |

**Example 8.** Tonic cosmetic water

| INGREDIENTS | Max. levels % (w/w) |
| --- | --- |
| Ethanol (alcohol denat.) | 70 |
| Humectants | 25 |
| Lingonberry extract | 0.01-20 |
| Oils (e.g. vegetable oils) | 50 |
| Additional ingredients | 5 |
| Emulsifying agents | 5 |
| Thickeners | 2 |
| Preservatives, antimicrobials | 2 |
| Perfume | 1.5 |
| Colorants | 1 |
| Aqua | to 100 |

In the above compositions, humectants include for example glycerin and propylene glycol. Additional ingredients include for example vitamins and UV filters. Emulsifying agents include anionic, amphoteric or non-ionic surfactants, for example long chain alcohols, polyglyceryl esters and sorbitan stearate. Thickeners include for example carbomer and xanthan gum.

**Example 9.** Lip care product or lip colour product (stick, cream or gel)

| INGREDIENTS | Max. levels % (w/w) |
| --- | --- |
| Oils, waxes and fats | 90 |
| Humectants | 50 |
| Aqua | 50 |
| Film forming agents | 30 |
| Thickeners | 15 |
| UV filters | 15 |
| Bulking agents (e.g. silica) | 10 |
| Emulsifying agents | 5 |

(continued)

| INGREDIENTS | Max. levels % (w/w) |
|---|---|
| Cosmetic colorants | 5 |
| Ethanol (alcohol denat.) | 5 |
| Lingonberry extract | 0.01-20 |
| Perfume | 2 |
| Preservatives, antimicrobials | 1 |
| Flavouring agents | 1 |

In the above composition, humectants include for example glycerin and propylene glycol. An example of film forming agents is VP/hexadecane copolymer. Typical thickeners in the above composition include for example carbomer and hydroxyethylcellulose. Examples of emulsifying agents include for example trioleyl phosphate and glyceryl dibehenate. Examples of suitable waxes include carnauba wax and candelilla cera wax. Further, the composition may also comprise vitamins and antioxidants.

**Example 10.** Cleansing gel

| INGREDIENTS | Max. levels % (w/w) |
|---|---|
| Anionic surfactants | 20 |
| Oils, waxes and fats | 20 |
| Humectants | 20 |
| Non-ionic / amphoteric surfactants | 15 |
| Foam boosting agents | 5 |
| Lingonberry extract | 0.01-20 |
| Thickeners | 5 |
| Preservatives, antimicrobials | 2 |
| Perfume | 1 |
| Colorants | 1 |
| Aqua | to 100 |

In the above composition, examples of anionic surfactants are sodium lauroyl sarcosinate and sodium laureth sulfate. Humectants include for example glycerine and propylene glycol. Non-ionic or amphoteric surfactants include for example betaine derivatives and glucose derivatives. An example of foam boosting agents is cocamide MEA (monoethanolamine). An example of suitable thickeners for the above composition is hydroxypropyl methylcellulose.

**Example 11.** Foundation

| INGREDIENTS | Max. levels % (w/w) |
|---|---|
| Isoparaffin and volatile emollients, like C15-19 alkanes and C13- alkanes | 60 |
| Oils | 40 |
| Emollients (e.g. plant esters) | 30 |
| Silicones | 30 |
| Bulking agents | 30 |
| Colorants, color additives | 30 |
| Humectants | 25 |
| Thickeners, emulsifying agents | 20 |
| Ethanol (alcohol denat.) | 15 |
| UV filters | 10 |
| Surfactants | 5 |
| Lingonberry extract | 0.01-20 |
| Perfume | 1 |
| Preservatives, antimicrobials | 1 |
| Aqua | to 100 |

In the above composition, suitable isoparaffins include for example branchedchain isoparaffin (C11-C16), isododecane and isohexadecane. Typical bulking agents include for example talc, kaolin, silica and starch. Colour additives may include for example opacifying agents and pearlescent agents. Surfactants may be anionic, amphoteric and/or non-ionic surfactants, such as sodium lauroyl sarcosinate.

**Example 12.** Mascaras

| INGREDIENTS | Max. lev % (w/w) |
|---|---|
| Silicones excl. volatile silicones | 80 |
| Oils, waxes and fats | 30 |
| Resins, film forming polymers | 30 |
| Colorants, colour additives | 25 |
| Emulsifying agents (e.g. stearates) | 20 |
| Bulking agents | 15 |
| Thickeners | 12 |
| Ethanol (alcohol denat.) | 10 |
| Aqua | 60 |
| Humectants | 5 |
| Lingonberry extract | 0.01-20 |
| Perfume | 1 |
| Antioxidants | 1 |
| Preservatives, antimicrobials | 1 |

A waterproof mascara

| INGREDIENTS | Max. levels % (w/w |
|---|---|
| Liquid paraffin and isoparaffin | 80 |
| Silicones excl. volatile silicones | 80 |
| Oils, waxes and fats | 30 |
| Resins, film forming polymers | 30 |
| Colorants, colour additives | 25 |
| Emulsifying agents (e.g. cera alba) | 20 |
| Bulking agents | 15 |
| Thickeners | 12 |
| Ethanol (alcohol denat.) | 10 |
| Aqua | 10 |
| Humectants | 5 |
| Lingonberry extract | 0.01-20 |
| Perfume | 1 |
| Antioxidants | 1 |
| Preservatives, antimicrobials | 1 |

In the above composition, suitable isoparaffins include for example branchedchain isoparaffin (C11-C16), isododecane and isohexadecane. Typical silicones include for example cyclopentasiloxane and dimethicone. Film forming polymers include for example aluminium distearate and VP/hexadecane copolymer. Colour additives may include for example opacifying agents and pearlescent agents. Typical bulking agents include for example talc, kaolin, and zinc stearate. An example of a suitable thickener for the above composition is stearalkonium hectorite. Usable waxes include natural waxes, such as bees wax, candelilla, carnauba, cereal based waxes, jojoba wax and their derivatives, berry fruit based waxes, birch based waxes (suberin), and sunflower seed wax.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

As will be understood from the preceding description of the present invention and the illustrative experimental examples, the present invention can be described by reference to the following embodiments:

1. A method of protecting human skin and keratin fibres from effects of high energy visible light comprising a wavelength of about 380 nm to about 530 nm, the method comprising topically applying on the skin or keratin fibres a cosmetic composition comprising

- lingonberry extract or lingonberry powder or both in an amount of 0.001% to about 50% by weight of the composition;
- a cosmetically acceptable carrier.

2. The method according to embodiment 1 wherein the amount of lingonberry extract or lingonberry powder or both is 0.001 to 20%, preferably from 0.007% to about 20%, more preferably from 0.01 to 20%, by weight of the composition.

3. The method according to embodiment 1 or 2, wherein the lingonberry extract or lingonberry powder comprises quercetin in an amount of 0.4-30 mg/g dry matter, preferably 1-30 mg/g dry matter.

4. The method according to any one of the preceding embodiments, wherein the lingonberry extract or lingonberry powder comprises polyphenolic compounds in an amount of 2-400 mg/g dry matter, preferably at least 10 mg/g dry matter, more preferably at least 20 mg/g dry matter.

5. The method according to any one of the preceding embodiments, wherein the lingonberry extract or lingonberry powder has an ORAC value of 200 - 7000 $\mu$mol TE/g.

6. The method according to any one of the preceding embodiments, wherein the lingonberry extract has been obtained by stepwise extraction of lingonberry fiber powder with one or more solvents, preferably solvents selected from ethanol and ethyl acetate.

7. The method according to any one of the preceding embodiments, wherein the lingonberry extract has been obtained by aqueous alcoholic extraction of lingonberry press cake, followed by chromatography to obtain an enriched extract, and optional spray-drying of the enriched extract to obtain spray-dried lingonberry extract powder.

8. The method according to any one of the preceding embodiments, wherein the lingonberry powder has been obtained by a method wherein lingonberry juice is pressed from lingonberry fruit, filtered and mixed with maltodextrin, and the mixture is spray-dried.

9. The method according to any one of the preceding embodiments wherein the cosmetically acceptable carrier comprises a liquid, a powder, or a wax, or more than one of these.

10. The method according to any one of the preceding embodiments, wherein the composition reduces the formation of reactive oxygen species (ROS) and reduces a risk of skin photo-aging and premature skin aging.

11. The method according to any one of the preceding embodiments, wherein the keratin fibres comprise human hair, eye lashes, eye brows, beard and moustache, in particular eye lashes.

12. The method according to any one of the preceding embodiments, wherein the cosmetic composition contains lingonberry extract, preferably spray-dried enriched extract.

13. A cosmetic composition for use in protecting human skin and keratin fibres from effects of high energy visible light, comprising:

- lingonberry extract or lingonberry powder or both in an amount of 0.001% to about 50% by weight of the composition;
- cosmetically acceptable carrier for applying the composition topically to skin or keratin fibres.

14. The cosmetic composition according to embodiment 13, which comprises lingonberry extract in an amount of 0.007 — 20 %, preferably 0.01 — 20 % by weight, wherein the lingonberry extract has a total concentration of phenolic compounds, which is at least 10 mg/g, preferably at least 20 mg/g.

15. The cosmetic composition according to embodiment 13 or 14, further comprising at least one of UVA/UVB filters, oils, waxes, fats, moisturizing agents, thickeners, vitamins, antioxidants, bulking agents, emulsifying agents, preserving agents, coloring agents, perfumes, film forming agents, foam boosting agents, emollients, and aqua.

16. The cosmetic composition according to any one of embodiments 13 to 15, wherein the cosmetic composition is selected from the group consisting of skin care cream, skin care lotion, skin care gel, skin softener, skin toner, ointment, milk lotion, moisture lotion, nutrition lotion, nutrition cream, emulsion cream, moisture cream, hand cream, skin serum, essence, nutrition essence, zinc oxide based cream (with sun protection factor), cosmetic tonic water, cleansing foam, cleansing lotion, cleansing cream, cleansing gel, body lotion, body cleanser, makeup base, foundation cream, pressed powder, loose powder, concealer, primer, mascara, lipstick, lip gloss, lip cream, lip gel, eye shadow, eye brow coloring products, make-up setting spray, beard and moustache cleansing and conditioning products, leave-on treatment for scalp and hair, and hair styling products.

17. The cosmetic composition according to any one of embodiments 13 to 16, wherein the composition contains oils and/or extracts from other Nordic plants in addition to lingonberry extract or lingonberry powder.

18. The cosmetic composition according to embodiment 17, wherein the oils and/or extracts comprise chaga extract, cloudberry seed oil and seabuckthorn berry oil.

19. Use of lingonberry extract or lingonberry powder for protecting human skin and keratin fibres from effects of high energy visible light.

INDUSTRIAL APPLICABILITY

At least some embodiments of the present invention find industrial application in cosmetic industry, particularly in the production of skin care compositions.

ACRONYMS LIST

| | |
|---|---|
| HEV | high energy visible |
| HEVPF | in vitro protection factor against HEV |
| IR | infrared |
| LED | light-emitting diode |
| NHEK | normal human epidermal keratinocytes |
| ORAC | oxygen radical absorbance capacity |
| ROS | reactive oxygen species |
| SSHI-HEV | % solar spectral irradiance of HEV blocked |
| TE | trolox equivalent |

CITATION LIST

Patent Literature

JP 2015044773 A

KR 102087868 A

US 20130078205 A1

WO 2006134583 A1

WO 2016176845 A1

Non Patent Literature

Hettwer S, Gyenge EB, Obermayer B (2017) Blue Light Protecting Cosmetic Active Ingredients: A Case Report. J Dermat Cosmetol 1(4)

Huang, D., Ou, B., Hampsch-Woodill, M., Flanagan, J.A. and Prior, R. L., High-Throughput assay of oxygen radical absorbance capacity (ORAC) using a multichannel liquid handling system coupled with a microplate fluorescence reader in 96-well format, J. Agric. Food Chem., 2002, 50, 4437-4444.

Magalhães, L. M., Santos, F., Segundo, M.A., Reis, S. and Lima, J.L.F.C. Rapid microplate high-throughput methodology for assessment of Folin-Ciocalteu reducing capacity, Talanta, 2010, 83, 441-447.

Nakashima Y, Ohta S, Wolf AM, Blue light-induced oxidative stress in live skin. Free Radic Biol Med, 2017 Jul; 108; 300-310.

**EP 3 964 194 A1**

Rascalou A, Lamartine J, Poydenot P, Demarne F, Bechetoille N, 2018. Mitochondrial damage and cytoskeleton reorganization in human dermal fibroblasts exposed to artificial visible light similar to screen-emitted light. Journal of Dermatological Science 91 (2018) 195-205.

**Claims**

1. A method of protecting human skin and keratin fibres from effects of high energy visible light comprising a wavelength of about 380 nm to about 530 nm, the method comprising topically applying on the skin or keratin fibres a cosmetic composition comprising

   - lingonberry extract or lingonberry powder or both in an amount of 0.001% to about 50% by weight of the composition; and
   - a cosmetically acceptable carrier;

   wherein the lingonberry extract and lingonberry powder comprise polyphenolic compounds in an amount of at least 10 mg/g dry matter, and quercetin in an amount of at least 0.4 mg/g dry matter.

2. The method according to claim 1 wherein the amount of lingonberry extract or lingonberry powder or both is 0.001 to 20%, preferably from 0.007% to about 20%, more preferably from 0.01 to 20%, by weight of the composition.

3. The method according to claim 1 or 2, wherein the lingonberry extract or lingonberry powder comprises quercetin in an amount of 0.4-30 mg/g dry matter, preferably 1-30 mg/g dry matter.

4. The method according to any one of the preceding claims, wherein the lingonberry extract or lingonberry powder comprises polyphenolic compounds in an amount of 10-400 mg/g dry matter, preferably at least 20 mg/g dry matter.

5. The method according to any one of the preceding claims, wherein the lingonberry extract or lingonberry powder has an ORAC value of 200 - 7000 $\mu$mol TE/g.

6. The method according to any one of the preceding claims, wherein the lingonberry extract has been obtained by stepwise extraction of lingonberry fiber powder with one or more solvents, preferably solvents selected from ethanol and ethyl acetate.

7. The method according to any one of the preceding claims, wherein the lingonberry extract has been obtained by aqueous alcoholic extraction of lingonberry press cake, followed by chromatography to obtain an enriched extract, and optional spray-drying of the enriched extract to obtain spray-dried lingonberry extract powder.

8. The method according to any one of the preceding claims, wherein the lingonberry powder has been obtained by a method wherein lingonberry juice is pressed from lingonberry fruit, filtered and mixed with maltodextrin, and the mixture is spray-dried.

9. The method according to any one of the preceding claims wherein the cosmetically acceptable carrier comprises a liquid, a powder, or a wax, or more than one of these.

10. The method according to any one of the preceding claims, wherein the composition reduces the formation of reactive oxygen species (ROS) and reduces a risk of skin photoaging and premature skin aging.

11. The method according to any one of the preceding claims, wherein the cosmetic composition contains lingonberry extract, preferably enriched lingonberry extract, preferably as spray-dried enriched lingonberry extract.

12. A cosmetic composition for use in protecting human skin and keratin fibres from effects of high energy visible light, comprising:

   - lingonberry extract or lingonberry powder or both in an amount of 0.001% to about 50% by weight of the composition;
   - cosmetically acceptable carrier for applying the composition topically to skin or keratin fibres,

wherein the lingonberry extract or lingonberry powder has a concentration of polyphenolic compounds, which is at least 10 mg/g dry matter, and a total concentration of quercetin, which is at least 0.4 mg/g dry matter.

13. The cosmetic composition according to claim 12, which comprises lingonberry extract in an amount of 0.007 — 20 %, preferably 0.01 — 20 % by weight, wherein the lingonberry extract has a total concentration of phenolic compounds, which is at least 20 mg/g, and a total quercetin concentration of at least 1 mg/g dry matter.

14. The cosmetic composition according to claim 12 or 13, wherein the composition contains oils and/or extracts from other Nordic plants in addition to lingonberry extract or lingonberry powder.

15. The cosmetic composition according to claim 14, wherein the oils and/or extracts are selected from chaga extract, cloudberry seed oil and seabuckthorn berry oil.

16. Use of lingonberry extract or lingonberry powder for protecting human skin and keratin fibres from effects of high energy visible light.

Fig. 1A

Fig. 1B

| In-Vitro Protection Factor | Percent Solar Spectral Irradiance Blocked (% SSI$_{HEV}$) |
|---|---|
| HEVPF = 6.65 | 85.0% |

Fig. 1C

| In-Vitro Protection Factor | Percent Solar Spectral Irradiance Blocked (% SSI$_{HEV}$) |
|---|---|
| HEVPF = 1.05 | 4.5% |

Fig. 1D

**HEV Light Transmisson Spectrum**
(3 PMMA Plates, 15 Scans Average)

%$\overline{HEVP}$ = 17.63%

Fig. 2

**LINGONBERRY FIBER POWDER**

Fig. 3

**EP 3 964 194 A1**

Europäisches
Patentamt

European
Patent Office

Office européen
des brevets

# EUROPEAN SEARCH REPORT

Application Number

EP 21 39 7509

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 2 914 242 A2 (LUMENE OY [FI]) 9 September 2015 (2015-09-09) | 1-6, 9-14,16 | INV. A61K8/9789 A61Q17/04 A61Q19/08 |
| Y | * paragraphs [0002], [0012], [0019], [0020] * * paragraphs [0057] - [0063]; examples 1-3 * * examples 4-16 * * claims * | 1-16 | |
| X,D | KR 102 087 868 B1 (KOLMAR KOREA CO LTD [KR]; DERMALAB [KR]) 11 March 2020 (2020-03-11) | 1-6, 9-14,16 | |
| Y | * paragraphs [0001], [0008], [0010], [0013], [0029] - [0030] * * paragraphs [0015], [0024] * * claims * * paragraphs [0068], [0072]; examples * | 1-16 | |
| X | US 2010/297272 A1 (LI CHUNHUA [CN] ET AL) 25 November 2010 (2010-11-25) * paragraphs [0002], [0025] * * paragraph [0103]; examples 1-3, 10-13; table 7 * * claims * | 1-5, 9-13,16 | |
| | | | TECHNICAL FIELDS SEARCHED (IPC) A61K A61Q |
| Y | CN 111 150 670 A (BEIJING BRILLIANCE BIOCHEMICAL CO LTD) 15 May 2020 (2020-05-15) * paragraphs [0029], [0034], [0035] * * example 2 * | 1-16 | |

-/--

The present search report has been drawn up for all claims

| Place of search | Date of completion of the search | Examiner |
|---|---|---|
| Munich | 20 January 2022 | Perrone Dunet, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

24

Europäisches
Patentamt

European
Patent Office

Office européen
des brevets

# EUROPEAN SEARCH REPORT

Application Number

EP 21 39 7509

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BAORU YANG ET AL: "Composition and antioxidative activities of supercritical CO-extracted oils from seeds and soft parts of northern berries", FOOD RESEARCH INTERNATIONAL, ELSEVIER, AMSTERDAM, NL, vol. 44, no. 7, 14 February 2011 (2011-02-14), pages 2009-2017, XP028098966, ISSN: 0963-9969, DOI: 10.1016/J.FOODRES.2011.02.025 [retrieved on 2011-02-20] * table 3 * | 1-16 | |
| Y | WO 2018/115582 A1 (TEKNOLOGIAN TUTKIMUSKESKUS VTT OY [FI]) 28 June 2018 (2018-06-28) * page 13, lines 37-41 * * page 24, line 10 - page 25, line 7 * | 1-16 | |
| Y | KIANTAMA: "Berry Powders and Crushed Berries", INTERNET CITATION, 1 January 2011 (2011-01-01), XP002764615, Retrieved from the Internet: URL:http://www.kiantama.fi/en/products/berry-powders-and-crushed-berries/ [retrieved on 2016-11-23] * the whole document * | 1-16 | **TECHNICAL FIELDS SEARCHED (IPC)** |
| Y | US 2008/095719 A1 (HERRMANN MARTINA [DE] ET AL) 24 April 2008 (2008-04-24) * claims 15, 19, 20 * | 1-16 | |

The present search report has been drawn up for all claims

| Place of search | Date of completion of the search | Examiner |
|---|---|---|
| Munich | 20 January 2022 | Perrone Dunet, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 21 39 7509

This annex lists the patent family members relating to the patent documents cited in the above-mentioned European search report.
The members are as contained in the European Patent Office EDP file on
The European Patent Office is in no way liable for these particulars which are merely given for the purpose of information.

20-01-2022

| Patent document cited in search report | | Publication date | Patent family member(s) | | Publication date |
|---|---|---|---|---|---|
| EP 2914242 | A2 | 09-09-2015 | DK | 2914242 T3 | 22-01-2018 |
| | | | EP | 2914242 A2 | 09-09-2015 |
| | | | ES | 2655562 T3 | 20-02-2018 |
| | | | FI | 124984 B | 15-04-2015 |
| | | | LT | 2914242 T | 12-02-2018 |
| | | | NO | 2914242 T3 | 12-05-2018 |
| | | | PL | 2914242 T3 | 30-04-2018 |
| | | | WO | 2014068190 A2 | 08-05-2014 |
| KR 102087868 | B1 | 11-03-2020 | NONE | | |
| US 2010297272 | A1 | 25-11-2010 | JP | 2010531816 A | 30-09-2010 |
| | | | US | 2010297272 A1 | 25-11-2010 |
| | | | WO | 2009003352 A1 | 08-01-2009 |
| CN 111150670 | A | 15-05-2020 | NONE | | |
| WO 2018115582 | A1 | 28-06-2018 | EP | 3558331 A1 | 30-10-2019 |
| | | | KR | 20190097244 A | 20-08-2019 |
| | | | WO | 2018115582 A1 | 28-06-2018 |
| US 2008095719 | A1 | 24-04-2008 | AT | 415976 T | 15-12-2008 |
| | | | EP | 1761271 A1 | 14-03-2007 |
| | | | JP | 4927718 B2 | 09-05-2012 |
| | | | JP | 2008502656 A | 31-01-2008 |
| | | | US | 2008095719 A1 | 24-04-2008 |
| | | | WO | 2005123101 A1 | 29-12-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016176845 A1
- US 20130078205 A1
- KR 102087868 A
- JP 2015044773 A
- WO 2006134583 A1
- EP 2914242 B1
- EP 291424 B1
- EP 2914242 A

### Non-patent literature cited in the description

- **HETTWER S ; GYENGE EB ; OBERMAYER B.** Blue Light Protecting Cosmetic Active Ingredients: A Case Report. *J Dermat Cosmetol,* 2017, vol. 1 (4
- **HUANG, D. ; OU, B. ; HAMPSCH-WOODILL, M. ; FLANAGAN, J.A. ; PRIOR, R. L.** High-Throughput assay of oxygen radical absorbance capacity (ORAC) using a multichannel liquid handling system coupled with a microplate fluorescence reader in 96-well format. *J. Agric. Food Chem.,* 2002, vol. 50, 4437-4444
- **MAGALHÃES, L. M. ; SANTOS, F. ; SEGUNDO, M.A. ; REIS, S. ; LIMA, J.L.F.C.** Rapid microplate high-throughput methodology for assessment of Folin-Ciocalteu reducing capacity. *Talanta,* 2010, vol. 83, 441-447
- **NAKASHIMA Y ; OHTA S ; WOLF AM.** Blue light-induced oxidative stress in live skin. *Free Radic Biol Med,* July 2017, vol. 108, 300-310
- **RASCALOU A ; LAMARTINE J ; POYDENOT P ; DEMARNE F ; BECHETOILLE N.** Mitochondrial damage and cytoskeleton reorganization in human dermal fibroblasts exposed to artificial visible light similar to screen-emitted light. *Journal of Dermatological Science,* 2018, vol. 91, 195-205